**Europäisches Patentamt**

**European Patent Office**

**Office européen des brevets**

(11) Veröffentlichungsnummer: **0 112 324**
**B1**

# (12) EUROPÄISCHE PATENTSCHRIFT

(45) Veröffentlichungstag der Patentschrift:
**18.01.89**

(21) Anmeldenummer: **83890192.4**

(22) Anmeldetag: **28.10.83**

(51) Int. Cl.⁴: **G 01 N 1/00, G 01 N 27/28**

(54) **Elektrochemische Messeinrichtung.**

(30) Priorität: **19.11.82  AT 4229/82**

(43) Veröffentlichungstag der Anmeldung:
**27.06.84 Patentblatt 84/26**

(45) Bekanntmachung des Hinweises auf die Patenterteilung:
**18.01.89 Patentblatt 89/3**

(84) Benannte Vertragsstaaten:
**DE FR GB**

(56) Entgegenhaltungen:
**US-A- 3 556 950**
**US-A- 3 611 790**
**US-A- 3 664 194**
**US-A- 4 298 026**

(73) Patentinhaber: **AVL AG, Grabenstrasse 11,**
**CH-8201 Schaffhausen (CH)**

(72) Erfinder: **Marsoner, Hermann, Jakominiplatz 17,**
**A-8010 Graz (AT)**
Erfinder: **Harnoncourt, Karl, Stiftingtalstrasse 71,**
**A-8010 Graz (AT)**
Erfinder: **List, Helmut, Bogengasse 36, A-8010 Graz (AT)**

(74) Vertreter: **Krause, Walter, Dr. Dipl.-Ing.,**
**Postfach 200 Singerstrasse 8, A-1010 Wien (AT)**

**Beschreibung**

Die Erfindung betrifft eine elektrochemische Messeinrichtung mit einer Messzelle, einer Zuführeinrichtung für ein Proben- und ein Reinigungsmedium und einer Beschickungseinrichtung zur Zuführung von Referenzmedien zur Messzelle, wobei die Beschickungseinrichtung eine mit Anschlüssen für die einzelnen Medien und mit einem Auslass zur Messzelle versehene Steuereinheit aufweist, welche aus einem starren Zuführteil und einem im Zuführteil drehbar gelagerten Drehelement gebildet ist, wobei von Anschlüssen ausgehende Zuführleitungen für Medien durch den Zuführteil über eine Umfangsöffnung im Drehelement in eine an den Auslass angeschlossene Durchlassleitung des Drehelements münden und durch Verdrehen des Drehelementes, wahlweise einzelne Anschlüsse mit dem Auslass verbindbar sind.

Bei der Durchführung von chemischen Analysen mit derartigen Messeinrichtungen, z.B. Blutgasanalysen, wird mit Hilfe von Messzellen mit Messfühlern bekannter Art, eine Kombination von Messwerten bestimmt. Dieses sind beispielsweise der pH-Wert der Blutprobe, weiters der Partialdruck des molekulargelösten Sauerstoffs und der Partialdruck von $CO_2$. Eine Messeinrichtung, in der elektrochemische Sensoren zur Messung der genannten drei Parameter eingesetzt werden können, ist beispielsweise im österreichischen Patent Nr. 349 151 beschrieben. Eine derartige Messrichtung wird üblicherweise in einer thermostatisierten metallischen Haltevorrichtung eingebaut. Diese Haltevorrichtung sorgt dafür, dass die Messkammer selbst auf einer gleichmässigen Temperatur, für Blutgasanalysen meist 37 °C, gehalten wird und das die messempfindlichen Spitzen der Fühler für die genannten drei Parameter an der Kontaktzone zu jenem Raum, der das Probenmaterial enthält, dicht abschliessen.

Die Messzelle muss über eine Einrichtung verfügen, die es erlaubt, Probenmaterialien verschiedener Art zuzuführen bzw. sie mit Referenzflüssigkeiten und/oder Referenzgasen zu beschicken.

Aus der US-PS 3 556 950 ist eine Einrichtung bekannt geworden welche ein Drehelement in Form eines konischen Stopfens aufweist, der in einer konischen Ausnehmung eines Zuführteils mit radial zugeführten Medienanschlüssen drehbar gelagert ist. Im konischen Stopfen befindet sich eine Durchlassleitung, welche einen den einzelnen Medienanschlüssen zuordenbaren radialen Abschnitt aufweist, der in einen axialen Abschnitt übergeht, welcher mit der Messzelle in Verbindung steht. Als Nachteil dieser Einrichtung ist die abgewinkelte Durchlassleitung im konischen Stopfen anzuführen, welcher nur in ungenügender Oberflächengüte herstellbar ist und dadurch Reinigungsprobleme aufwirft. Ausserdem neigt der konische Stopfen dazu, Probenmaterial längs seiner Oberfläche bzw. der konischen Ausnehmung des Zuführteils zu verschleppen und auch hier eine von der Reinigungslösung nicht erfassbare Zone zu schaffen.

Das Problem der Abdichtung zwischen Zuführteil und Drehelement ist zum Teil durch eine aus der US-PS 4 298 026 bekannten Vorrichtung gelöst, wo ein zylindrischer Stopfen verwendet wird, dessen zahlreiche Durchlassleitungen mittels O-Ringen gegen die Anschlüsse im Zuführteil abgedichtet sind. Die genannten Reinigunsschwierigkeiten treten jedoch auch beim relativ kompliziert aufgebauten Drehelement dieser Vorrichtung zu Tage.

In einer weiteren bekannten Einrichtung der genannten Art wird ein Drehelement verwendet, das in Form einer kreisförmigen Scheibe ausgeführt ist und eine Reihe von auf einem Kreis angeordnete Öffnungen enthält, die mit der Zuleitung der Messzelle durch eine entweder händisch oder automatisch gesteuerte Drehbewegung in Verbindung gebracht werden können. Auf der der Messzelle abgewandten Seite des drehbaren Elements können Verbindungsschläuche zu Reagenzienbehältern sowie zu Gasbehältern angeschlossen werden. Weiters wird die Messkammer über dieses bekannte Drehelement mit einem Behälter von Reinigungslösung und einem Abfallbehälter verbunden, in den die Probenreste durch Evakuieren des letzteren abgesaugt werden. Dabei wird gleichzeitig die Reinigungslösung nachgesaugt. Nach Abschluss des Reinigungsvorganges wird das drehbare Element in eine solche Position gebracht, dass Aussenluft nachgesaugt wird, mit der die Messzelle wieder getrocknet wird.

Die Probe kann bei der bekannten Einrichtung ebenfalls in einer bestimmten Position des drehbaren Elements an diesem selbst eingegeben werden. Dazu wird an einer geeignet dafür ausgebildeten Stelle eine Glaskapillare, die die Probe enthält, angesteckt. In dieser Position des Drehelements ist der Ausgang der Messzelle mit einer peristaltischen Pumpe verbunden, die die Probe langsam in die Messzelle einsaugt. Der Ausgang der peristaltischen Pumpe führt zum Abfallbehälter.

Diese bekannte Einrichtung weist eine Reihe von Nachteilen auf: Das Drehelement muss in allen Anschlusspositionen gegenüber der Messzelle dicht anschliessen. Bei der Drehbewegung werden die Dichtelemente, die aus elastischem Material sein müssen, beansprucht, so dass ein gewisser Abrieb entsteht, dessen Partikel die dünnen Verbindungsbohrungen von und zur Messzelle blockieren können. Weiters bedingt dieser Abrieb, dass die Verbindung vom drehenden Element zur Messzelle undicht wird, was zu Betriebsstörungen führt.

Auch hier entsteht im Drehelement eine Zone, die nach abgeschlossener Probenaufnahme nicht gereinigt werden kann. Dadurch entsteht beispielsweise bei Messungen von Blut eine unhygienische Zone, die durch geronnenes Blut blockiert werden kann.

Die vorliegende Erfindung hat die Aufgabe, die genannten Nachteile der bisher bekannten elektrochemischen Messeinrichtungen zu vermeiden und eine mechanisch einfache, dichte, leicht zu reinigende Einrichtung zur Zufuhr von Proben-, Referenz- und Reinigungsmedien vorzuschlagen.

Dies geschieht gemäss der vorliegenden Erfindung dadurch, dass das Drehelement als ein die Durchlassleitung bildendes gerades zylindrisches Rohr ausgebildet ist, welches an einem Ende an die Zuführreinrichtung für ein Proben- und ein Reinigungsmedium und am anderen Ende an die Messzelle über drehbare Abdichtungen angeschlossen ist und das im Bereich der Umfangsöffnung am Rohr Dichtungen zur Abdichtung gegen die Zuführleitungen im Zuführteil vorgesehen sind.

Durch die Ausbildung des Drehelements als gerades zylindrisches Rohr ist zuverlässig sichergestellt, dass innerhalb der Messeinrichtung nach Einbringen der Probe in die Messzelle und anschliessender Reinigung keine Probenreste verbleiben können, wodurch deren Funktion sicher gewahrt bleibt. Aufgrund der erfindungsgemässen Ausgestaltung des Zuführteils und des Drehelementes sind auch die bei den genannten bekannten Einrichtungen aufgetretenen Nachteile hinsichtlich der Abdichtung der Beschickungseinrichtung vermieden, wobei die nur sehr kleine erforderliche Dichtfläche im Bereich der Umfangsbohrung des Drehelementes sehr vorteilhaft ist.

Obwohl es prinzipiell auch möglich wäre, zur Steuerung der Medienanschlüsse eine relative Verschiebung zwischen Zuführteil und Drehelement vorzusehen, ist die beschrieben Verdrehung in konstruktiver Hinsicht wesentlich einfacher zu realisieren, wobei durch den sehr präzise steuerbaren Schrittmotor eine schnelle und exakte Änderung der jeweils mit der Messzelle zu verbindenden Medienanschlüsse möglich ist.

In diesem Zusammenhang ist es natürlich vorteilhaft, wenn die Mündungen der Zuleitungen der einzelnen Medienanschlüsse in die Bohrung des Zuführteils jeweils in zumindest einer Ebene senkrecht zur Achse der Bohrung liegen, da in diesem Falle für jede derartige Ebene nur eine abgedichtete Umfangsbohrung am Drehelement vorgesehen sein muss.

Obwohl durch geeignete Werkstoffwahl und entsprechend gewählte Toleranzen auch das Drehelement selbst unmittelbar gegen die Bohrung im Zuführteil ausreichend dicht gemacht werden könnte, ist es doch in konstruktiver Hinsicht vorteilhaft, wenn im Bereich der Umfangsbohrung am Drehelement Dichtungen zur Abdichtung gegen die Mündungen im Zuführteil angeordnet sind.

Die Erfindung wird im folgenden anhand des in der Zeichnung dargestellten Ausführungbeispieles näher erläutert:

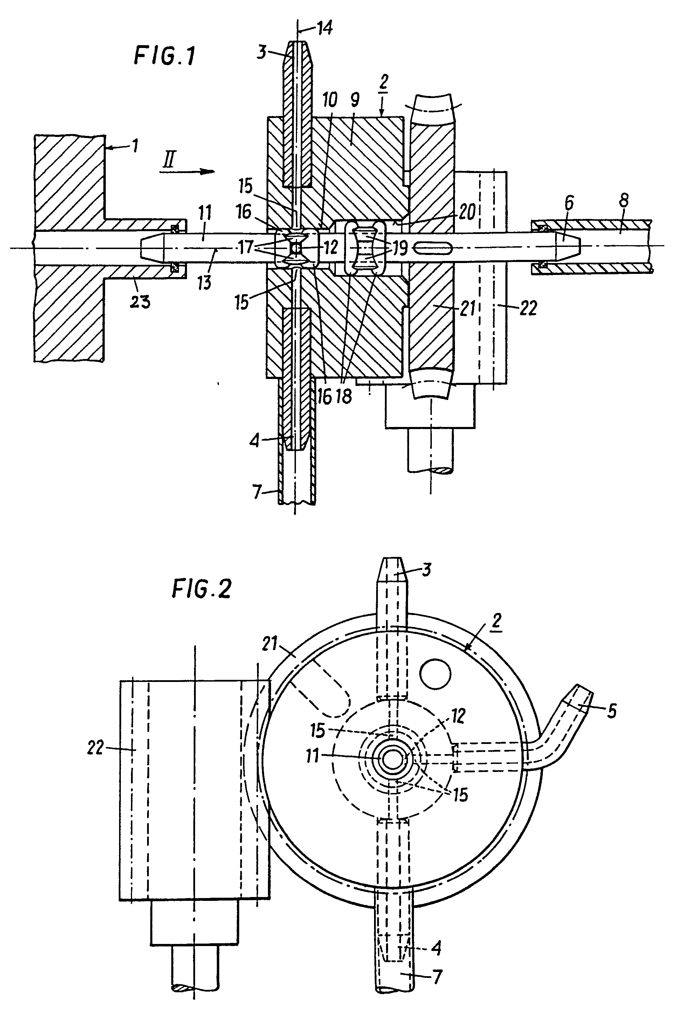

Figur 1 zeigt einen Schnitt durch eine erfindungsgemässe elektrochemische Messeinrichtung.

Figur 2 zeigt eine Ansicht entlang des Pfeiles II in Fig. 1.

Die teilweise nur schematisch dargestellte Einrichtung zur Beschickung einer elektrochemischen Messzelle 1 wahlweise mit Proben-, Referenz- bzw. Reinigungsmedien weist eine Steuereinheit 2 auf, an der Anschlüsse 3, 4, 5, 6 für die einzelnen Medien vorgesehen sind, wobei in der Darstellung nur die Anschlüsse 4 und 6 mit weiterführenden Schläuchen bzw. Rohren 7, 8 versehen sind. Die Steuereinheit 2 weist einen Zuführteil 9 sowie ein in einer Bohrung 10 des Zuführteiles 9 abdichtend angeordnetes und relativ zum Zuführteil 9 in der Bohrung 10 drehbar gelagertes Drehelement 11 auf, welches mit einer Umfangsbohrung 12 versehen ist. Das Drehelement ist als gerades, zylindrisches Rohr ausgebildet.

Die Anschlüsse 3, 4, 5, welche beispielsweise in hier nicht dargestellter Weise mit zur Justierung bzw. zum Betrieb der an sich in ihrer Funktion für die vorliegende Erfindung nicht weiter wichtigen elektrochemischen Messeinrichtung bzw. Messzelle erforderlichen Referenz- oder Puffermedien versorgt sind, liegen im dargestellten Ausführungsbeispiel mit ihren Achsen in einer senkrecht zur Achse 13 der Bohrung 10 liegenden Ebene 14, wodurch die Mündungen der Zuführleitungen 15 der einzelnen Medienanschlüsse bei Drehung des Drehelementes 11 um die Achse 13 nacheinander von der Umfangsbohrung 12 überschliffen werden. Um die Umfangsbohrung 12 sind im dargestellten Ausführungsbeispiel eigene Dichtlippen 16, 17 angebracht, welche beispielsweise von aufvulkanisierten Gummiwülsten gebildet sein können. Ähnliche Gummiwülste 18, 19 sind auch in einem Abstand von der Umfangsbohrung 12 rechts davon auf dem Drehelement 11 in einer Erweiterung 20 der Bohrung 10 vorgesehen, wobei sie dort allerdings nur zur Führung des zylindrischen Rohres gegenüber der Bohrung dienen.

Der an dem der Messeinrichtung abgewandten Ende des Drehelementes 11 vorgesehene Anschluss 6 dient direkt zur Zuführung der Probe sowie nach Beendigung der Probenzuführung zur Zuführung von Reinigungs- und Trockenmedium, womit auf alle Fälle sichergestellt ist, dass der innerhalb der Beschickungseinrichtung verlaufende Probenweg leicht und zuverlässig zu reinigen ist und keine den Betrieb der Beschickungseinrichtung störenden Ablagerungen auftreten können.

Zur Durchführung der für das wahlweise Öffnen jeweils einer der Zuführleitungen 15 durch die Umfangsbohrung 12 erforderlichen Relativverdrehung zwischen Steuereinheit 2 und Drehelement 11 ist auf letzterem ein Schneckenrad 21 mitdrehend befestigt, welches über eine Schnecke 22 von einem hier nicht dargestellten Schrittmotor kontrolliert verdrehbar ist. Das Drehelement 11 ist zu diesem Zwecke an seinen Enden einerseits in der Zuleitung 23 zur nicht weiter dargestellten elektrochemischen Messzelle 1 der Messeinrichtung und andererseits im Schlauchstück 8 der Zufuhreinrichtung für die Proben- bzw. Reinigungsmedien drehbar abgedichtet.

Es ist mit der dargestellten Einrichtung also beispielsweise möglich, zu Beginn einer Messung mit der Messzelle 1 Referenzmedium über einen der Anschlüsse 3, 4, 5 zuzuführen, wobei zu diesem Zweck das Drehelement 11 mittels des Schneckenrades 21 so verdreht ist, dass die entsprechende Zuführleitung 15 mit der Umfangsbohrung 12 zusammenwirkt; der Schlauch 8 ist dabei über ein hier nicht dargestelltes Abschlussorgan, z.B. ein Schlauchklemmventil, geschlossen. Sodann kann beispielsweise durch Verdrehung des Drehelementes 11 um 90°C einer der anderen in der Ebene 14 liegenden Anschlüsse in Zusammenwirkung mit der Umfangsbohrung 12 gebracht werden, was etwa die Zuführung eines weiteren Referenzmediums oder eines Puffermediums ermöglicht. Sodann kann die Umfangsbohrung 12 durch Verdrehen des Drehelementes 11 in eine mit keiner der Zuführleitungen 15 zusammenarbeitende Stelle gebracht werden – z.B. die der Mündung des Anschlusses 5 gegenüberliegende Stelle – womit – nach Öffnung des genannten Absperrorganes im Schlauch 8 – beispielsweise von dort Probe zuströmen kann. Nach Abschluss der Messung und anschliessender Reinigung des Probenzuflussweges kann der beschriebene Vorgang – beispielsweise zu Einleitung einer Messung von anderen Bestandteilen der Probe – wieder beginnen.

**Patentansprüche**

1. Elektrochemische Messeinrichtung mit einer Messzelle (1), einer Zuführeinrichtung für ein Proben- und ein Reinigungsmedium und einer Beschickungseinrichtung zur Zuführung von Referenzmedien zur Messzelle (1), wobei die Beschickungseinrichtung eine mit Anschlüssen (3, 4, 5, 6) für die einzelnen Medien und mit einem Auslass zur Messzelle (1) versehene Steuereinheit (2) aufweist, welche aus einem starren Zuführteil (9) und einem im Zuführteil (9) drehbar gelagerten Drehelement (11) gebildet ist, wobei von Anschlüssen (3, 4, 5) ausgehende Zuführleitungen für Medien durch den Zuführteil (9) über eine Umfangsöffnung (12) im Drehelement (11) in eine an den Auslass angeschlossene Durchlassleitung des Drehelements (11) münden und durch Verdrehen des Drehelementes (11), wahlweise einzelne Anschlüsse (3, 4, 5) mit dem Auslass verbindbar sind, dadurch gekennzeichnet, dass das Drehelement (11) als ein die Durchlassleitung bildendes gerades zylindrisches Rohr ausgebildet ist, welches an einem Ende an die Zuführeinrichtung für ein Proben- und ein Reinigungsmedium und am anderen Ende an die Messzelle (1) über drehbare Abdichtungen angeschlossen ist und dass im Bereich der Umfangsöffnung (12) am Rohr (11) Dichtungen (16, 17) zur Abdichtung gegen die Zuführleitungen (15) im Zuführteil (9) vorgesehen sind.

2. Elektrochemische Messeinrichtung nach Anspruch 1, dadurch gekennzeichnet, dass die Zuführleitungen (15) im Zuführteil (9) in einer Ebene (14) senkrecht zur Achse (13) des Drehelementes (11) liegen.

**Claims**

1. An electrochemical measuring apparatus comprising a measuring cell (1), a feeder device for a sample medium and a cleansing medium, and a charging device for feeding reference media into the measuring cell (1), the said charging device being provided with a control unit (2) with connection (3, 4, 5, 6) for the individual media and an outlet towards the measuring cell (1), which control unit (2) is made up of a rigid feeder part (9) and a rotating element (11) rotatably mounted in the said feeder part (9), where feeder lines for the media beginning at the connections (3, 4, 5) and passing the feeder part (9), will open into a through-pipe of the rotating element (11) – which pipe is connected to the above outlet – via a circumferential opening (12) in the rotating element (11), and where any of the connections (3, 4, 5) may be connected to the outlet by turning the rotating element (11), wherein the rotating element (11) ist configured as a straight, cylindrical pipe acting as through-pipe which, on one end, is connected to the feeder device for a sample medium and a cleansing medium, and, on its other end, is connected to the measuring cell (1) via rotatable sealings, and wherein sealings (16, 17) are provided in the area of the circumferential opening (12) on the pipe (11) for the purpose of establishing a seal against the feeder lines (15) in the feeder part (9).

2. An electrochemical measuring apparatus according to claim 1, wherein the feeder lines (15) in the feeder part (9) are situated in a plane (14) normal to the axis (13) of the rotating element (11).

**Revendications**

1. Appareil électro-chimique de mesure avec une cellule de mesure (1), avec un dispositif d'amenée pour un produit d'échantillonnage et un produit de nettoyage, et avec un dispositif d'alimentation pour amener des produits de référence à la cellule de mesure (1), ce dispositif d'alimentation comportant une unité de mesure (2) munie de raccords (3, 4, 5, 6) pour les différents produits et munie d'une évacuation vers la cellule de mesure (1), cette unité de mesure étant constituée par une pièce rigide d'amenée (9) et un élément rotatif (11) monté de façon à pouvoir tourner dans cette pièce d'amenée (9), tandis que des canalisations d'amenée pour les produits, partant des raccords (3, 4, 5), débouchent, à travers la pièce d'amenée (9) et par l'intermédiaire d'un orifice périphérique (12) dans l'élément rotatif (11), dans une canalisation de passage, raccordée à l'évacuation, de l'élément rotatif (11), et par rotation de cet élément rotatif (11) différents raccords (3, 4, 5) étant susceptibles d'être reliés à volonté à l'évacuation, appareil électro-chimique de mesure caractérisé en ce que l'élément rotatif (11) est réalisé sous la forme d'un tube cylindrique rectiligne constituant la canalisation de passage, ce tube, par l'intermédiaire de joints d'étanchéité suspectibles de tourner, étant raccordé à une extrémité au dispositif d'amenée pour un produit d'échantillonnage et un produit de nettoyage, tandis qu'à son autre extrémité, il est raccordé à le cellule de mesure (1), cependant que dans la zone de l'orifice périphérique (12) sur le tube (11) il est prévu des joints d'étanchéité (16, 17) pour assurer l'étanchement vis à vis des canalisations d'amenée (15) dans la pièce d'amenée (9).

2. Appareil électro-chimique de mesure selon la revendication 1, caractérisé en ce que les canalisations d'amenée (15), dans la pièce d'amenée (9), se situent dans un plan (14) perpendiculaire à l'axe (13), de l'élément rotatif (11).

FIG.1

FIG.2